(19) **Europäisches Patentamt**
**European Patent Office**
**Office européen des brevets**

(11) **EP 2 689 000 B1**

(12) **EUROPEAN PATENT SPECIFICATION**

(45) Date of publication and mention
of the grant of the patent:
**16.03.2016 Bulletin 2016/11**

(21) Application number: **12711380.1**

(22) Date of filing: **16.03.2012**

(51) Int Cl.:
*C11D 3/37* *(2006.01)*    *C08L 33/14* *(2006.01)*
*C08F 220/28* *(2006.01)*    *A61K 8/81* *(2006.01)*
*A61Q 1/02* *(2006.01)*    *A61Q 3/00* *(2006.01)*
*A61Q 5/00* *(2006.01)*    *A61Q 5/02* *(2006.01)*
*A61Q 5/06* *(2006.01)*    *A61Q 15/00* *(2006.01)*
*A61Q 17/04* *(2006.01)*    *A61Q 19/02* *(2006.01)*
*A61Q 19/10* *(2006.01)*    *C08F 220/06* *(2006.01)*
*C08F 220/18* *(2006.01)*    *C08F 222/28* *(2006.01)*

(86) International application number:
**PCT/EP2012/054720**

(87) International publication number:
**WO 2012/130644 (04.10.2012 Gazette 2012/40)**

(54) **PERSONAL CARE COMPOSITIONS**

KÖRPERPFLEGEZUSAMMENSETZUNGEN

COMPOSITIONS DE SOINS PERSONNELS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.03.2011 IT VA20110008
28.09.2011 IT VA20110027**

(43) Date of publication of application:
**29.01.2014 Bulletin 2014/05**

(73) Proprietor: **Lamberti SpA
21041 Albizzate (VA) (IT)**

(72) Inventors:
• **FUMAGALLI, Chiara
I-21010 Besnate (VA) (IT)**

• **BALDARO, Eva
I -20127 Milano (IT)**
• **FLORIDI, Giovanni
I-28100 Novara (IT)**
• **LI BASSI, Giuseppe
I-21026 Gavirate (VA) (IT)**

(74) Representative: **Giaroni, Paola
LAMBERTI S.p.A.
Ufficio Brevetti
Via Piave, 18
21041 Albizzate (VA) (IT)**

(56) References cited:
**US-A- 4 529 773    US-A1- 2005 131 176**

## Description

### Technical field

The present invention relates to personal care compositions and in particular to aqueous thickened compositions for use in personal care comprising as thickeners and suspending agents crosslinked alkali swellable polyacrylates containing one or more acetoacetyl or cyanoacetyl groups. The crosslinked alkali swellable polyacrylates containing one or more acetoacetyl or cyanoacetyl groups possess high thickening capability in the presence of surfactants and electrolytes, provide homogeneous and clear solutions and give excellent performances in terms of suspending properties.

### Background art

It is known that a technical problem often encountered in the detergent & toiletries industries is to obtain stable viscous homogeneous formulations comprising surfactants, electrolytes and possibly dispersed solid particles. The thickeners employed shall develop their thickening capability without negatively altering the other properties of the formulations.

Most often, thickeners are also asked to control the whole rheology of the compositions, including the yield value.

In the field of aqueous personal care thickened compositions, the thickeners are generally selected among crosslinked polyacrylic acids and crosslinked acrylic acid copolymers (belonging to the class of crosslinked alkali swellable polyacrylates), guar gum and its derivatives, starch and its derivatives, crosslinked polyoxyethylene, carboxymethylcellulose, partially hydrolyzed crosslinked water swellable polymers such as partially hydrolyzed polyacrylamides, associative polyurethanes.

Crosslinked polyacrylic acids and crosslinked acrylic acid copolymers partially neutralized to the sodium salts are often preferred.

In the specialised literature many methods are reported to regulate the rheological properties of different formulations including the use of crosslinked acrylic acid copolymers performing as thickeners at neutral and basic pH.

We cite as an example:

- GB 870,994, disclosing copolymers of methacrylic acid and $C_1$-$C_4$ alkyl acrylate in aqueous emulsion;
- EP 13836, describing thickeners based on copolymers of (meth)acrylic acid, $C_1$-$C_4$ (meth)acrylic esters, selected (meth)acrylic esters comprising polyoxyethylene units, and optionally polyethylenically unsaturated monomers; the thickeners are said to be less sensitive to electrolytes than prior art synthetic thickeners that are prepared without the use of the selected (meth)acrylic esters;
- US 4,529,773 and US 4,138,381, describing alkali-soluble emulsion acrylic polymers comprising associative monomers and a method for their use;
- US 6,140,438, describing crosslinked acrylic polymers possessing high thickening and suspending properties obtainable by polymerization of acrylic monomers in the presence of specific crosslinking agents;
- EP 1272159, describing the use of crosslinked acrylate copolymers generally free from, i.e. containing less than about 1% by weight, associative monomers.

### Summary of the disclosure

In accordance with the present disclosure personal care compositions are prepared comprising:

i) from 0.2 to 10 % by weight, preferably from 0.5 to 8 % by weight, of a thickening agent which is a crosslinked alkali swellable polyacrylate obtainable by polymerization of:

a) from 20 to 70% by weight, preferably from 20 to 50 % by weight, of a monoethylenically unsaturated monomer containing a carboxylic group;
b) from 20 to 70% by weight, preferably from 40 to 70 % by weight, of a (meth)acrylic acid ester;
c) from 0.05 to 3% by weight, preferably from 0.1 to 2 % by weight, of an unsaturated monomer containing one or more acetoacetyl or cyanoacetyl groups;
d) from 0.01 to 3% by weight, preferably from 0.02 to 1 % by weight, of a polyethylenically unsaturated monomer;
e) from 0 to 10% by weight of a nonionic acrylic associative monomer;

ii) from 0.1 to 60% by weight, preferably from 0.5 to 20 % by weight, more preferably from 5 to 20% by weight, of a surfactant selected from anionic surfactants, amphoteric surfactants, cationic surfactants, zwitterionic surfactants, non-ionic surfactants, and mixture thereof.

In the personal care compositions, the surfactants may act as detergent agents, emulsifiers, co-emulsifiers, humectants, thickeners, foaming agents, dispersants or solubilizers.

The above described crosslinked alkali swellable polyacrylates have proved to efficiently perform as thickeners in the presence of electrolytes and surfactants and possess improved suspending and thickening properties in comparison with crosslinked alkali swellable polyacrylates of the prior art.

**Detailed description of the disclosure**

With the expression "personal care compositions" we mean the products normally used for personal detergence (such as: shampoos in general, and in particular 2-in-1 shampoos, baby shampoos, conditioning shampoos, moisturizing shampoos, temporary hair color shampoos, 3-in-1 shampoos, anti-dandruff shampoos, hair color maintenance shampoos, acid or neutralizing shampoos, salicylic acid shampoos; skin and body cleansers in general, and in particular shower gels, bath foams, facial cleansers, intimate cleansers, moisturizing body washes, antibacterial body washes; bath gels; hand soaps; bar soaps; body scrubs; bubble baths; facial scrubs; foot scrubs), hair care compositions (such as hair dyes, hair conditioners, hair creams and hair styling formulations), skin care compositions (such as skin lotions, balms and creams in general, and in particular alpha-hydroxy acid lotions and creams, beta-hydroxy acid creams and lotions, skin whiteners, self tanning lotions, sunscreen lotions, barrier lotions, moisturizers, vitamin C creams, antibacterial lotions and other moisturizing lotions and creams, liquid talc products, skin gels, for example facial masks, body masks, hydroalcoholic gels, body gels, sunscreen gels, make up foundations, sun care formulations, antiperspirants) and personal care products performing more than one of the above functions.

A key point in the preparation of the thickener of the present invention is the presence, among the monomers of the alkali swellable crosslinked polyacrylate, of an unsaturated monomer containing one or more acetoacetyl or cyanoacetyl groups.

The acetoacetyl and cyanoacetyl groups respectively have the formula $-CO-CH_2-COCH_3$ and $-CO-CH_2-CN$.

Examples of useful unsaturated monomers containing one or more acetoacetyl or cyanoacetyl groups are acetoacetoxyalkyl (meth)acrylates, allyl acetoacetates, vinyl acetoacetates, unsaturated acetoacetamides, allylcyanoacetates.

Preferably the unsaturated monomer containing one or more acetoacetyl or cyanoacetyl groups is an unsaturated monomer containing one or more acetoacetyl group.

More preferably the unsaturated monomer containing one or more acetoacetyl groups is an acetoacetoxyalkyl (meth)acrylate, and in particular an acetoacetoxyalkyl (meth)acrylate of formula

$$CH_2=CR-CO-O-R_1-O-CO-CH_2-CO-CH_3,$$

in which R is H or $CH_3$ and $R_1$ is a $C_2-C_4$ linear or branched alkylene radical group.

Most preferably the unsaturated monomer containing one or more acetoacetyl groups is acetoacetoxyethyl methacrylate, i.e. an acetoacetoxyalkyl (meth)acrylate) of formula

$$CH_2=CR-CO-O-R_1-O-CO-CH_2-CO-CH_3$$

in which R is $CH_3$ and $R_1$ is the ethylene radical.

Acetoacetoxyethyl methacrylate has a reactive methylene group that is useful for crosslinking and modifying the polymer after its synthesis.

The reactive methylene group is known to react with melamines, isocyanates, Michael reactive double bonds, aldehydes.

Beside the reactive methylene group, acetoacetoxyalkyl (meth)acrylates contain a reactive carbonyl group, which also undergoes reactions that are useful for crosslinking or modification of resins.

The carbonyl group of acetoacetylated polymers is in equilibrium between its enol and keto forms: enolization enables chelation of the acetoacetylated polymer with polyvalent cations from metals, such as zinc, tin, aluminum, copper, zirconium, and reaction with amines and diamines.

Because of this peculiar chemistry, acetoacetoxyethyl methacrylate finds use as an acrylic monomer for preparing post reaction crosslinkable polymers for coatings, plastics and adhesive applications.

By way of example, US 5,516,453, describes a stable ambient curing one-pack composition containing an active methylene-functional component and an active methylene-reactive component that may be used as coating, adhesive and impregnating agent and that can be applied on a variety of substrates, such as wood, cement and concrete, fabrics, metals, ceramics, plastics.

Beside the unsaturated monomer containing reactive methylene groups, the crosslinked alkali swellable polyacrylates of the disclosure are obtained by co-polymerization of one or more monoethylenically unsaturated monomers containing a carboxylic group, one or more (meth)acrylic esters, one or more polyethylenically unsaturated monomer and, possibly, one or more nonionic acrylic associative monomer.

Acrylic acid, methacrylic acid, itaconic acid and mixtures thereof are examples of monoethylenically unsaturated monomer containing a carboxylic group that are useful for the preparation of the crosslinked alkali swellable polyacrylates of the present disclosure.

Methacrylic acid is the preferred monoethylenically unsaturated monomer containing a carboxylic group.

Useful (meth)acrylic acid esters are $C_1$-$C_{40}$ (meth)acrylic acid alkyl ester, such as methyl, ethyl, propyl, butyl, 2-ethylhexyl, lauryl (meth)acrylates and mixtures thereof.

Preferably the (meth)acrylic acid ester is ethyl acrylate.

The addition of the polyethylenically unsaturated monomer, acting as polymer crosslinker, is a further key point in the preparation of the thickeners of the present invention.

The polyethylenically unsaturated monomer can be any of the known polyfunctional derivatives that are known to undergo radical polymerization with (meth)acrylic monomers.

Among the useful polyethylenically unsaturated monomer we cite diallyl maleate, allyl methacrylate, diallyl phthalate, N-methylene-bis-acrylamide, pentaerithritol ether polyacrylates, triallyl cianurate.

The nonionic acrylic associative monomer may be selected among (meth)acrylic acid esters of $C_8$-$C_{30}$ alkyl, alkylaryl or polycyclic hydrocarbyl monoether of a polyethylene glycol having at least two oxyethylene units, preferably having 10 to 40 oxyethylene units, and having up to 70 oxyethylene units, this ester having general formula

$$H_2C=C(R)\text{-}CO\text{-}O(CH_2CH_2O)_n\text{-}R',$$

wherein

R is H or $CH_3$, the latter being preferred,
n is at least 2, and preferably has an average value of at least 10, up to 40 to 60 or even up to 70 or so, and R' is a hydrophobic group, for example an alkyl, alkylaryl, or polycyclic alkyl group having 8 to 30 carbon atoms, preferably 16 to 18 carbon atoms, or more preferably having an average of 12 to 18 carbon atoms.

Other unsaturated monomers may be used in the polymerization beside the monomers a) to e), such as, by way of example, other nonionic acrylic monomers, monoethylenically unsaturated monomers, possibly containing a sulfonic acid group, cationic acrylic monomers.

Non limiting examples of other utilizable unsaturated monomers are vinyl acetate, styrene, vinyl chloride, vinylidene chloride, acrylonitrile, (meth)acrylamide, N,N,-dimethyl(meth)acrylamide, t-butyl-(meth)acrylamide, sodium vinyl sulfonate, 2-acrylamido-2-methylpropane sulfonic acid.

Nonetheless, by using only the monomers from a) to e) in polymerization, excellent thickening and suspending performances of the crosslinked alkali swellable polyacrylates in aqueous detergent compositions are obtained. The crosslinked alkali swellable polyacrylates of the disclosure can also indirectly be obtained by reaction of the monomers a), b), d) and possibly e), in the presence of an unsaturated monomer comprising a hydroxyl group and post synthesis reaction of the obtained polymer with commercially available t-butyl acetoacetate.

The crosslinked alkali swellable polyacrylate has high Brookfield® viscosity at pH 7.5 in water, i.e. has Brookfield® viscosity (spindle 6, RVT, 20 rpm, 1.0% by weight and 20 °C) of at least 500 mPa·s.

According to an embodiment of the disclosure, the surfactant of the personal care compositions of the invention is anionic or a mixture of anionic surfactants.

Anionic surfactants include alkyl sulfates, alkyl ether sulfates, alkyl sulfonates, alkylamido sulfonates, alkylaryl sulfonates, alkylamido ether sulfates, alkylaryl polyether sulfates, monoglycerides sulfates, alpha-olefin sulfonates, paraffin sulfonates, alkyl and alkylaryl phosphates, alkyl ether and alkylaryl ether phosphates, alkyl sulfosuccinates, alkyl ether sulfosuccinates, alkylamide sulfosuccinate, alkylsulfoacetate, alkylsarcosinates, acylglutamate, alkyl carboxylates, fatty acids salts (soaps), fatty acyllactylates, alkyl ether carboxylates, anionic derivatives of alkyl polyglycosides, such as the citric, tartaric or sulfosuccinic ester of alkyl polyglucosides.

The amphoteric or zwitterionic surfactants which can be used in the compositions of the present disclosure are those which can be broadly described as derivatives of aliphatic amines or quaternary ammonium compounds, containing an anionic water-solubilizing group, e.g., a carboxylate, sulfonate, sulfate, phosphate, phosphonate group. Examples of amphoteric or zwitterionic surfactants include cocoamphocarboxypropionate, disodium lauroamphodiacetate, disodium caprylamphodiacetate, disodium cocoamphodipropionate, disodium lauroamphodipropionate, disodium caprylamphodipropionate, cocoamphocarboxy propionic acid, sodium cocoamphoacetate, disodium cocoamphodiacetate, sodium lauroamphoacetate, lauroamphodipropionic acid, cocoamphodipropionic acid, ($C_8$-$C_{20}$)alkyl betaines and

4

$(C_8-C_{20})$alkylamido$(C_6-C_8)$ alkylbetaines, sultaines, $(C_8-C_{20})$alkylamido$(C_6-C_8)$ alkylsulfobetaines alkyl glycinates and alkyl carboxyglycinates.

Cationic surfactants useful in the compositions of the present disclosure contain amino or quaternary ammonium moieties which are positively charged when dissolved in an aqueous composition of the present invention. Examples of cationic surfactants are long-chain alkyl trimethyl ammonium chlorides, long-chain alkyl benzyl dimethyl ammonium chlorides, alkylamine hydrochlorides, alkylamine acetates and di(long-chain alkyl) dimethyl ammonium bromides.

In the case of anionic, cationic or amphoteric surfactants, the counter-ion can be monoatomic or polyatomic, inorganic or organic. Examples of counterions are: alkali metal, alkaline earth metal, transition metal, chloride ($Cl^-$), bromide ($Br^-$), iodide ($I^-$), ammonium, pyridinium, triethanolaminium, methylsulfate.

Nonionic surfactants can be broadly defined as compounds containing a hydrophobic moiety and a nonionic hydrophilic moiety. Examples of the hydrophobic moiety can be alkyl, alkyl aromatic and aryl aromatic. Examples of hydrophilic moieties are polyoxyalkylenes, amine oxides, hydroxyl groups and alkanol amides. Examples of non ionic surfactants are fatty alcohols, alkoxylated fatty alcohols, alkoxylated fatty acids, glycerol alkyl esters, alkoxylated di- and tri-stiryl phenols, polyhydroxy fatty acid amides, sugar esters and polyesters, alkoxylated sugar esters, sorbitan and alkoxylated sorbitan fatty acid esters, block copolymers of polyethylene glycol and polypropylene glycol. Other examples of nonionic emulsifiers include alkyl polyglycosides, such as coco polyglucosides.

Surprisingly, the crosslinked alkali swellable polyacrylates of the disclosure containing reactive methylene groups show an enhanced Brookfield® viscosity in water in the presence of surfactants. A wide range of surfactant type and amount is effective.

Although it is known from the prior art that the introduction of specific associative monomers in crosslinked alkali swellable polyacrylates may enhance the thickening performance in the presence of surfactants, the remarkable enhancement of thickening that has been observed upon the addition of a surfactant to an aqueous system containing the thickeners of the invention is independent from the presence of nonionic acrylic associative monomers in the polymer. This phenomenon is not observed in the same way with analogous crosslinked alkali swellable polyacrylates of the prior art that do not contain acetoacetyl or cyanoacetyl groups. What more, it has been found that even little amounts of monomer containing reactive methylene groups, significantly improve the suspending properties of aqueous detergent compositions.

According to one of the preferred embodiments of the disclosure, to enhance the suspending properties of the thickeners, from 0.1 to 0.5 wt % of the unsaturated monomer containing one or more acetoacetyl or cyanoacetyl groups is used in polymerization, together with 0.1 to 2%, more preferably 1.0 to 2.0 wt%, of the nonionic acrylic associative monomer. Advantageously, the reactive methylene group of the thickeners of the aqueous liquid detergent compositions of the disclosure may further provide tunable viscosifying properties to the polymer in the presence of several common additives of detergent compositions, such as preservatives (in particular formaldehyde or formaldehyde donors) and polyvalent metal cations. The amount and kind of both the reactive methylene groups in the polymer and the additives may serve to tune the viscosity of the final liquid aqueous compositions, as the person skilled in the art would easily determine with few experiments.

The crosslinked alkali swellable polyacrylates of the present disclosure may be prepared in any conventional manner, such as for instance, by precipitation polymerization, suspension and solution polymerization, emulsion polymerization. Generally, the crosslinked alkali swellable polyacrylates of the present disclosure are prepared by emulsion polymerization.

The emulsion polymerization techniques are well known in the art such as, for example, as disclosed in U.S. Patents No. 4,325,856 ; 4,654,397 ; and 4,814,373. Conventional surfactants may be used in emulsion polymerization, such as anionic and/or nonionic emulsifiers, for example, alkali metal or ammonium alkyl sulfates, alkyl sulfonic acids, fatty acids, and oxyethylated alkyl phenols. The amount of surfactant used is usually 0.1% to 6% by weight, based on the weight of total monomer. Thermal or redox initiation processes may be used. Conventional free radical initiators may be used such as, for example, hydrogen peroxide, t-butyl hydroperoxide, t-amyl hydroperoxide, alkali or ammonium persulfates, and azo initiators such as 4,4'-azobis(4-cyanopentanoic acid), and 2,2'-azobisisobutyronitrile ("AIBN"), typically at a level of 0.01% to 3.0% by weight, based on the weight of total monomer. Redox systems using the same initiators coupled with a suitable reductant such as, for example, sodium sulfoxylate formaldehyde, sodium hydrosulfite, isoascorbic acid, hydroxylamine sulfate and sodium bisulfite may be used at similar levels, optionally in combination with metal ions such as, for example iron and copper, optionally further including complexing agents for the metal. Chain transfer agents such as mercaptans may be used to lower the molecular weight of the polymers. The monomer mixture may be added neat or as an emulsion in water. The monomer mixture may be added in a single addition or in multiple additions or continuously over the reaction period using a uniform or varying composition. The emulsion polymerization process may utilize a preformed seed emulsion polymer such as, for example, by adding 5% (based on total monomer) of the monomer mixture to the kettle and making it react previously. Techniques to reduce residual monomer such as, for example, subjecting the reaction mixture to steam stripping, hold times, and additional radical sources may be employed.

The crosslinked alkali swellable polyacrylates of the present disclosure are generally supplied in their acidic form, in emulsion or in solid form; as they contain acidic groups, they need to be neutralized to the salt form to develop optimal viscosity increase in the personal care compositions.

In many cases the personal care compositions have neutral pH values and the compositions of the disclosure may be prepared by simple dilution of the acidic emulsion of the crosslinked alkali swellable polyacrylates or by dissolution of the crosslinked alkali swellable polyacrylates in solid form in a nearly neutral aqueous solution, accompanied by the addition of the surfactant and of any optional ingredient.

Surfactants, which are essential ingredients of the personal care compositions, can be added after or before the crosslinked alkali swellable polyacrylates, but are preferably added after the crosslinked alkali swellable polyacrylates; if needed, for the neutralization of the crosslinked alkali swellable polyacrylates, high diffusion alkalis are commonly used, such as sodium or potassium hydroxide, ethanolamine, ammonia, etc.. Subsequently, the pH of the obtained thickened composition may also be lowered, when and how advisable, without significant impairment of the viscosity level; typically weak organic acids, such as citric acid, salicylic acid and the like may be used.

Additional ingredients of the personal care compositions of the disclosure are those commonly known in the art and, by way of example, may be selected among silicones and water insoluble oily compounds, cationic polymers, cosmetically acceptable mediums, other natural or synthetic thickeners, skin and hair care actives, particulate materials, perfumes, fragrances, essential oils, dyes, pearlescent agents, opacifiers, scrubbing agents, enzymes, preservatives, disinfecting agents, antimicrobial additives, foaming agents, anti-foam agents, humectants, moisturizing agents, chelating agents, emollients, conditioning agents, whitening agents, solvents and pH buffering means.

According to an embodiment of the disclosure, the personal care compositions additionally comprise at least one silicone and/or water insoluble oil (oily compounds).

Silicone and oily compounds are often incorporated into personal care compositions for conditioning, especially hair and skin, and to improve or impart shine, gloss, water resistance or lubricity. These materials can also function as moisture barriers or protectants.

The silicone to be used according to this disclosure can be insoluble or soluble in water.

Suitable water-insoluble, non-volatile silicones include amodimethicone, amodimethicone macroemulsions or microemulsions, polyalkylsiloxanes, poly-arylsiloxanes, polyalkylarylsiloxanes, polysiloxane gums, and polyethersiloxane copolymers. Preferred are high molecular weight polydimethylsiloxanes and other silicone materials such as dimethiconol, phenyldimethicone, polymethylphenyl polysiloxanes, organopolysiloxanes, alkoxysilicones, polydiorganosiloxanes, polydimethylsiloxane copolymers, and polyaminofunctional silicone. Water-insoluble silicones may also be considered oily conditioning agents.

Additional non-soluble, silicones which can be utilized include volatile silicones, for example cyclomethicone, or low viscosity polydimethylsiloxane.

Suitable water-soluble silicones include polyether/polysiloxane block copolymers, such as dimethicone copolyols, and derivatives thereof.

The most preferred silicones used as an oily conditioning agent are polydimethylsiloxanes which have the CTFA designation of dimethicone, dimethiconol, and emulsions thereof.

Other suitable oily compounds to be used in the personal care compositions of the invention include, but are not limited to the following: mineral oils and saturated or unsaturated vegetable oils such as soybean oil, babassu oil, castor oil, cottonseed oil, Chinese tallow oil, crambe oil, perilia oil, Danish rapeseed, rice bran oil, palm oil, palm kernel oil, olive oil, linseed oil, coconut oil, sunflower oil, safflower oil, peanut oil, corn oil, sesame oil, and avocado oil, as well as petrolatum; d-limonene, and esters such as isopropylpalmitate, cetearyl octanoate, C12-15 alkylbenzoate, octyl stearate, and other materials such as PPG-2 myristyl etherpropionate, and the like.

The silicone or the oily compound, or the combinations thereof, are present in a concentration of about 0.1% to about 20% by weight, more preferably of about 0.3% to about 7% by weight, and most preferably of about 0.5% to about 5% by weight on the composition.

According to an embodiment of the disclosure, the personal care compositions additionally comprise at least one cationic polymer, useful for improving the cosmetic properties of skin or hair.

As disclosed herein, the therm "cationic polymer" means any polymer comprising at least one group that may be ionized into a cationic group. Among the cationic polymers that mey be mentioned are, for example, polymers of the polyamine, polyamino amide and polyquaternium type. Among the polymers, exemplary mention may be made of:

- Acrylic copolymers and homopolymers;
- Cellulose ether derivatives comprising quaternary ammonium groups;
- Cationic guar derivatives;
- Polymers of alkyldiallylamine or of dialkyldiallylammonium;
- Polyquaternary ammonium polymers;
- Quaternary polymer of vinylpirrolidone and vinylimidazole.

Examples of suitable cationic polimers are known under the INCI names: Polyquaternium-5, Polyquaterium-7, Polyquaternium-47, Polyqaternium-57, Polyquaternium -10, Guar Hydroxypropyltrimonium Chloride, Hydroxypropyl Guar Hydroxypropyltrimonium Chloride Polyquaternium-39, Polyquaternium-22, Polyquaternium-6, Polyquaternium-2, Polyquaternium-76, Polyquaternium 74, Polyquaternium-11, Polyquaternium-16, Polyquaternium -44, Polyquaternium-68, Cetrimonium Chloride, Behentrimonium Chloride, Steartrimonium Chloride, Acrylamidopropyl Trimonium Chloride/Acrylamide Copolymer.

The at least one cationic polymer may be present in the composition disclosed herein in a total amount ranging from 0.01% to 5% by weight relative to the total weight of the composition.

The personal care compositions of the present disclosure may further comprise a cosmetically acceptable medium.

By "cosmetically acceptable medium" we mean a cosmetically acceptable solvent chosen from $C_1$-$C_4$ alcohols, such as ethanol, isopropanol, tert-butanol and n-butanol; polyols such as glycerol, propylene glycol and polyethylene glycols.

The pH of the compositions disclosed herein may range from 3 to 11, such as from 5 to 10 and preferably from 7 to 10.

The compositions disclosed herein may also comprise one or more natural or synthetic thickeners other than the crosslinked alkali swellable polyacrylates of the disclosure.

The thickener(s) other than the crosslinked alkali swellable polyacrylates may be chosen from synthetic thickeners such as crosslinked acrylic acid and acrylamidopropanesulfonic acid homopolymers, for example Carbomer, anionic, cationic and amphoteric associative polymers, such as the polymers sold under the names PEMULEN (R) TRI or TR2, SALCARE (R) SC90, ACULYN (R) 22, 28, 33, 44 or 46, and ELFACOS (R) T210 and T212. Associative hydrophobically modified polyurethane thickeners may also be used.

Examples of natural or modified natural polymers include but are not limited to gums (e.g. xanthan gum), modified cellulosics, starches, or polysaccharide and polysaccharide derivatives.

The compositions of the present invention may further comprise at least one skin or hair care active ("actives").

Classes of suitable actives include, but are not limited to sunscreens, vitamins, peptides and peptide derivatives, sugar amines, oil control agents, flavonoid compounds, antioxidants, preservatives, phytosterols, protease inhibitors, tyrosinase inhibitors, anti-inflammatory agents, and mixtures thereof.

Herein, "sunscreen" is understood to include both sunscreen actives and UV light absorbers. The sunscreen may be organic or inorganic, and may be water-soluble, oil-soluble, a particulate material which is insoluble in either an oil or an aqueous phase, or a mixture thereof. In one embodiment the compositions of the present invention comprise a water-soluble and an oil-soluble sunscreen.

Particularly suitable sunscreen actives are known under the following INCI names: benzophenone-3, benzophenone-4, Bis-Ethylhexyloxyphenol Methoxyphenyl Triazine, Butil Methoxydibenzoylmethane, Diethylamino Hydroxybenzoil Hexyl benzoate, Disodium Phenyl Dibenzimidazole Tetrasulfonate, Drometrizole Trisiloxane, Methylene Bis-benzotriazolyl Tetaramethylbutylphenol, Terephthalylidene Dicamphor Sulfonic Acid, Zinc Oxide, 4- Methylbenzylidene Camphor, Polysilicone-15, Diethylhexyl Butamido Triazone, Ethylhexyl Dimethyl PABA, Ethylhexyl Methoxycinnamate, Ethylhexyl Salycilate, Ethylhexyl Triazone, Homomenthyl Salicylate, Isoamyl p-Methoxycinnamate, Octocrylene, Phenylbenzimidazole Sulfonic Acid, Titanium Dioxide, and mixtures thereof. Herein, "vitamins" means vitamins, pro-vitamins, and their salts, isomers and derivatives. The vitamins may include water soluble vitamins, for example, vitamin B compounds (including B3 compounds such as niacinamide; nicotinic acid, C1-C18 nicotinic acid esters, and nicotinyl alcohol; B6 compounds, such as pyroxidine; and B5 compounds, such as panthenol, or "pro-B5"); and vitamin C compounds, including ascorbyl esters of fatty acids, and ascorbic acid derivative, and mixtures thereof. The vitamins also may include those exhibiting limited solubility in water, such as vitamin A compounds, and all natural and/or synthetic analogs of Vitamin A, including retinoids, carotenoids, and other compounds which possess the biological activity of Vitamin A; vitamin D compounds; vitamin E compounds, or tocopherol, including tocopherol sorbate, tocopherol acetate, other esters of tocopherol; vitamin K compounds; and mixtures thereof. In one embodiment, the compositions of the instant invention may comprise from about 0.0001% to about 10% by weight of the vitamin.

The compositions of the present invention may comprise one or more peptides, for example, to aid in repair of skin, to aid in exfoliation, and to deliver other benefits to the skin.

Herein, "peptide" refers to peptides containing ten or fewer amino acids, their derivatives, isomers, and complexes with other species such as metal ions (for example, copper, zinc, manganese, and magnesium). As used herein, peptide refers to both naturally occurring and synthesized peptides. In one embodiment, the peptides are di-, tri-, tetra-, penta-, and hexapeptides, their salts, isomers, derivatives, and mixtures thereof.

The compositions of the present invention may comprise a sugar amine, also known as amino sugars, and their salts, isomers, tautomers and derivatives. Sugar amines can be synthetic or natural in origin and can be used as pure compounds or as mixtures of compounds (e.g., extracts from natural sources or mixtures of synthetic materials).

The compositions of the present invention may comprise one or more compounds useful for regulating the production of skin oil, or sebum, and for improving the appearance of oily skin.

Examples of suitable oil control agents include salicylic acid, dehydroacetic acid, benzoyl peroxide, vitamin B3 compounds (for example, niacinamide), their isomers, esters, salts and derivatives, and mixtures thereof.

The compositions of the present invention may comprise a flavonoid, for example, to provide anti-oxidation benefits. The flavonoid can be synthetic materials or obtained as extracts from natural sources, which also further may be derivatized.

The compositions of the present invention may comprise non-vitamin antioxidants, preservatives, phytosterols and/or plant hormones, protease inhibitors, tyrosinase inhibitors, antiinflammatory agents and N-acyl amino acid compounds.

The compositions of the present invention may comprise a particulate material.

In one embodiment, the compositions may comprise from about 0.5% to about 10% of a particulate material, and alternatively from about 1% to about 5% of a particulate material.

Suitable particulate materials include, but are not limited to almond meal, aluminum oxide, apricot seed powder, bismuth oxychloride, boron nitride, cellulose and cellulose derivatives, clay, calcium oxide, inorganic salts, for example salts of carbonates and chlorides, iron oxide, jojoba seed powder, mica, peach pit powder, pecan shell powder, polyethylene, polybutylene, polyisobutylene, polymethylstyrene, polypropylene, polystyrene, polyurethane, nylon, polytetrafluoroethylene, polyhalogenated olefins, pumice, rice bran, sericite, silk, synthetic hectorite, titanium dioxide, tricalcium phosphate, and mixtures thereof. Also useful are particles made from mixed polymers (e.g., copolymers, terpolymers, etc.)

The polymeric and mixed polymeric particles can also optionally be cross linked with a variety of common crosslinking agents.

Other examples of useful particles include waxes and resins such as paraffins, carnauba wax, ozokerite wax, candellila wax, and ureaformaldehyde resins.

When such waxes and resins are used herein it is important that these materials are solids at ambient and skin temperatures.

Other examples of particulate materials useful in the present invention include colored and uncolored pigments, interference pigments, inorganic powders and organic powders other than those described above, composite powders, optical brightener particles, and mixtures thereof. The average size of such particulates in general may be smaller than the aforementioned particulate materials, ranging for example from about 0.1 microns to about 100 microns and can be added to the current compositions as a powder or as a pre-dispersion.

**EXAMPLES.**

The compositions of the Examples from 1 to 8 are model compositions comprising water, the crosslinked alkali swellable polyacrylate of the invention or a comparative crosslinked alkali swellable polyacrylate and a surfactant.

**Examples 1-6**

The thickened liquid aqueous detergent compositions of the Examples 1-6 were prepared according to the following procedure.

The crosslinked alkali swellable polyacrylates emulsion is prepared by emulsion polymerization and dispersed in deionized water at 2.7 wt% a.m.. Then, the detergent component is added (10.7 wt % a.m.) and after mixing the pH is adjusted to 6.6-6.8 by addition of NaOH.

The solution is further mixed by stirring for about 30 minutes, and allowed to stand overnight before the viscosity is measured.

Table 1 a describes the surfactant names and chemical compositions used in the Examples 1 to 6, while Table 1 b describes the monomer composition of the two crosslinked alkali swellable polyacrylates tested (PACI and PAC2); the quantities of monomers are in parts by weight (pbw).

The surfactants of Example 1 and 4 to 6 are commercialized by Zschimmer & Schwarz, while the surfactants of Examples 2 and 3 are commercialized by Lamberti SpA.

The viscosities of the thus obtained thickened liquid aqueous detergent compositions were measured using a Brookfield(R) Model LVT Viscometer at 25°C and at the indicated spindle speed, and are reported in Table 2 and Table 3 (BV).

**Table 1a - Surfactant**

| Examples | Trade name | Chemical description |
|---|---|---|
| Example 1 | ZETESOL LES 2/SL | Laureth (2EO) sulphate sodium salt 27 wt% a.m. (unpreserved) |

(continued)

| Examples | Trade name | Chemical description |
|---|---|---|
| Example 2 | ROLPON 24/230* | Laureth (2EO) sulphate sodium salt 27 wt% a.m. |
| Example 3 | ROLPON LS | Sodium lauryl sulphate 30 wt% a.m. |
| Example 4 | SULFETAL LA | Ammonium lauryl sulphate 27 wt% a.m. |
| Example 5 | ZETESOL LA-370 | Ammonium laureth(3EO) sulphate salt, 70 wt% a.m. |
| Example 6 | SETACIN 103 Spezial | Disodium laureth sulfosuccinate, 40 wt% a.m. |

**Table 1b - Polyacrylates**

| Monomer | PAC1* | PAC2 |
|---|---|---|
| Ethyl acrylate (pbw) | 62 | 62 |
| Methacrylic acid (pbw) | 38 | 38 |
| Acetoacetoxyethyl methacrylate (pbw) | - | 1.00 |
| Diallylmaleate (pbw) | 0.05 | 0.05 |
| *comparative | | |

**Table 2 - BV (mPa*s) and BYV**

| | Example 1 | | Example 2 | | Example 3 | |
|---|---|---|---|---|---|---|
| | BV | | | | | |
| rpm | PAC1* | PAC2 | PAC1* | PAC2 | PAC1* | PAC2 |
| 0.5 | 14480 | 29000 | 68000 | 380000 | 19800 | 568000 |
| 1 | 8840 | 18000 | 46400 | 254000 | 11000 | 300000 |
| 5 | 3248 | 5400 | 16280 | 197000 | 4000 | 136000 |
| 10 | 2172 | 3470 | 10860 | 133000 | 2750 | 90000 |
| 20 | 1510 | 2245 | 7430 | 80000 | 1850 | 52000 |
| 50 | 1042 | 1350 | 1044 | 40000 | 1150 | 25400 |
| 100 | 758 | 950 | 757 | 23000 | 785 | 14720 |
| | BYV | | | | | |
| | 56 | 110 | 220 | 1260 | 88 | 2680 |
| * comparative | | | | | | |

**Table 3 - BV (mPa*s) and BYV**

| | Example 4 | | Example 5 | | Example 6 | |
|---|---|---|---|---|---|---|
| | BV | | | | | |
| rpm | PAC1* | PAC2 | PAC1* | PAC2 | PAC1* | PAC2 |
| 0.5 | 21000 | 350000 | 33000 | 40000 | 36000 | 320000 |
| 1 | 13600 | 300000 | 22000 | 25000 | 22000 | 220000 |
| 5 | 4500 | 180000 | 6700 | 7400 | 6700 | 78000 |
| 10 | 2850 | 132000 | 4280 | 4670 | 4160 | 53000 |

(continued)

|  |  | Example 4 | | Example 5 | | Example 6 | |
|---|---|---|---|---|---|---|---|
|  |  | BV | | | | | |
| rpm | PAC1* | PAC2 | PAC1* | PAC2 | PAC1* | PAC2 |
| 20 | 1940 | 85000 | 2750 | 3000 | 2700 | 31000 |
| 50 | 1171 | 42000 | 1590 | 1728 | 1510 | 15000 |
| 100 | 830 | 24000 | 1130 | 1240 | 1086 | 8500 |
|  |  | BYV | | | | | |
|  | 74 | 500 | 110 | 150 | 140 | 1000 |
| *comparative | | | | | | | |

A measure of the pseudoplasticity of the compositions is the Brookfield Yield Value (BYV), which is calculated from the following formula :

$$BYV = (BV_{0.5\,rpm} - BV_{1\,rpm})/100$$

The BYVs are also reported in Table 2 and Table 3.

**Example 7**

The thickened liquid aqueous detergent compositions of Example 7 were prepared according to the following procedure.

The crosslinked alkali swellable polyacrylates were prepared by emulsion polymerization and dispersed in deionized water at 2.7 wt% a.m. and at 2.25 wt% a.m..

The surfactant is added (10.7 wt% a.m. of Sodium Laureth Sulfate and 2.4 wt% a.m. of Cocoamidopropyl Betaine) and mixed, then the pH is adjusted to 6.6-6.8 by addition of NaOH.

The solutions are mixed by stirring for about 30 minutes and allowed to stand overnight before the viscosity is measured.

Four crosslinked alkali swellable polyacrylates having the monomer compositions reported in Table 4 (PAC3, PAC4, PAC5, PAC6) were tested; the quantities of monomers are in parts by weight (pbw).

The viscosities (BV, mPa*s) and BYVs of the thus obtained thickened liquid aqueous detergent compositions were measured using a Brookfield(R) Model LVT Viscometer at 25°C and at the indicated spindle speed and are reported in Table 5 (2.7 wt% polyacrylate a.m.) and Table 6 (2.25 wt% polyacrylate a.m.) .

The thickened liquid aqueous detergent compositions containing PAC3, PAC4 and PAC5 are uncoloured and crystal clear, while the thickened liquid aqueous detergent composition containing PAC6 has light blue reflexes.

**Table 4 - Polyacrylates**

| Monomer | PAC3 | PAC4 | PAC5 | PAC6* |
|---|---|---|---|---|
| Ethyl acrylate (pbw) | 62 | 62 | 62 | 62 |
| Methacrylic acid (pbw) | 38 | 38 | 38 | 38 |
| polyethoxylated $C_{16}$-$C_{18}$ alcohols methacrylates (20EO) (pbw) | 1.7 | 1.7 | 1.7 | 1.7 |
| Acetoacetoxyethyl methacrylate (pbw) | 0.1 | 0.2 | 0.3 | - |
| Diallylmaleate (pbw) | 0.05 | 0.05 | 0.05 | 0.05 |
| *comparative | | | | |

**Table 5 - BV (mPa*s) and BYV (2.7 wt% a.m.)**

| BV | | | | |
|---|---|---|---|---|
| rpm | PAC3 | PAC4 | PAC5 | PAC6* |
| 0,5 | 42600 | 58400 | 512000 | 32000 |
| 1 | 30000 | 44400 | 288000 | 21600 |
| 5 | 15000 | 19120 | 108000 | 7750 |
| 10 | 8080 | 14600 | 71300 | 5700 |
| 20 | 5740 | 10750 | 44600 | 3750 |
| 50 | 3880 | 7260 | 25280 | 2330 |
| 100 | 2960 | 5520 | 16320 | 1638 |
| BYV | | | | |
| | 126 | 140 | 2240 | 104 |
| *comparative | | | | |

**Table 6 - BV (mPa*s) and BYV (2.25 wt% a.m.)**

| BV | | | | |
|---|---|---|---|---|
| rpm | PAC3 | PAC4 | PAC5 | PAC6* |
| 0,5 | 20320 | 31200 | 62800 | 18000 |
| 1 | 13280 | 18500 | 49000 | 12000 |
| 5 | 6040 | 6950 | 31800 | 4000 |
| 10 | 4280 | 4760 | 22800 | 2600 |
| 20 | 3120 | 3340 | 13400 | 1810 |
| 50 | 2184 | 2240 | 7140 | 1090 |
| 100 | 1710 | 1650 | 4600 | 800 |
| BYV | | | | |
| | 70 | 127 | 138 | 60 |
| * comparative | | | | |

It should be noted that the shear rate, as indicated herein by spindle speed (rpm), is an important parameter in viscosity measurement and that all the compositions according to the disclosure are noticeably pseudoplastic, or shear thinning.

This behaviour and the associated BYVs reflect the suspending properties of the composition, which are sensibly improved.

**Example 8**

The thickened liquid aqueous detergent compositions of Example 8 were prepared according to the following procedure.

The crosslinked alkali swellable polyacrylate (PAC4, see Table 4) was prepared by emulsion polymerization and dispersed in deionized water at 1.5 wt% a.m..

The surfactant is added (10.7 wt% a.m. of Sodium Laureth Sulfate and 2.4 wt% a.m. of Cocoamidopropyl Betaine) and mixed, then the pH is adjusted to 6.6-6.8 by addition of NaOH.

The amount of electrolyte (NaCl) reported in Table 7 (wt%) is added.

The solution was mixed by stirring for about 30 minutes and allowed to stand overnight before the viscosity is measured.

Five compositions with different amount of NaCl were tested.

The viscosities (BV, mPa*s) of the thus obtained thickened liquid aqueous detergent compositions were measured using a Brookfield(R) Model LVT Viscometer at 25°C and at the indicated spindle speed and are reported in Table 7.

All compositions were perfectly colorless and clear.

Table 7 - BV (mPa*s) (1.5 wt% a.m.)

| rpm | BV | | | | |
|-----|---------|-----------|-----------|-----------|-----------|
|     | 0% NaCl | 0.5% NaCl | 1.0% NaCl | 1.5% NaCl | 2.0% NaCl |
| 0,5 | 6000 | 6800 | 14880 | 26640 | 44400 |
| 1 | 4900 | 5120 | 11840 | 21200 | 34400 |
| 5 | 2500 | 2920 | 7024 | 13600 | 22240 |
| 10 | 1900 | 2280 | 5780 | 11720 | 18520 |
| 20 | 1625 | 2000 | 4865 | 10500 | 15760 |
| 50 | 1100 | 1472 | 4184 | 8740 | 13260 |
| 100 | 900 | 1382 | 3600 | 7500 | 12000 |

Examples 9-28 provide finished personal care compositions comprising a crosslinked alkali swellable polyacrylates prepared according to the invention (PAC4, see Table 4 for its composition), water, one or more surfactants and one or more further customary additives.

The personal case compositions of Examples 9-28 were prepared by mixing the listed ingredients, as reported below.

For the specific ingredients, the INCI names are reported.

All percentages are in weight and refer to the active matter of the ingredient.

**Example 9**

Salicylic Acid Antidandruff Shampoo

Phase A: Aqua (Water) to 100%, Acrylates Copolymer (PAC4) 1.5%, Sodium Laureth Sulfate 6.6%, Sodium Hydroxide to pH 6.5-7.0, Cocamidopropyl Betaine 3.6%

Phase B: Aqua (Water) 15%, Sodium Laureth Sulfate 4.5%, Glycerin 2% Salicylic Acid 2.0%

Phase C: Aqua (Water) 5%, Mica 0.2%, Preservative, Fragrance 1.0%, Polysorbate 20 2.0%, Sodium Chloride 0.2%, Citric Acid to pH 5

**Example 10**

Scrubbing Shower Gel

Phase A: Aqua (Water) to 100%, Tetrasodium EDTA 0.1%, Acrylates Copolymer (PAC4) 2.5%, Sodium Laureth Sulfate 9.0%, Sodium Hydroxide to pH 6.5-7.0, Glycerin 2.0%, Cocamidopropyl Betaine 1.26%

Phase B: Polysorbate 20 0.8%, Fragrance 0.6%

Phase C: Preservative 0.5%, Aqua (water) 5.0, Kaolin 0.2%

**Example 11**

Shower Milk

Phase A: Aqua (Water) to 100%, Acrylates Copolymer (PAC4) 2.5%, Sodium Laureth Sulfate 6%, Sodium Coco-Glucoside Tartrate 1.5%, Sodium Hydroxide to pH 6.5-7.0, Cocamidopropyl Betaine 1.5%

Phase B: Sunflower Seed Oil 10%, Cyclopentasiloxane 3.0%, Ethylhexyl Palmitate 5.0%

Phase C: Preservative, Fragrance, Citric Acid Solution to pH 5.5

**Example 12**

Clear Body gel

Phase A: Aqua (Water) to 100%, Acrylates Copolymer (PAC4) 2.0%, Sodium Coco-Glucoside Tartrate 1.5%, Triethanolamine to pH 6.5-7.0, Cocamidopropyl Betaine and Sodium Lauroyl Lactylate 9%, Phantenol 0.5%, Aloe Barbadensis Leaf Juice 0.5%, Fragrance

**Example 13**

Body Wash

Phase A: Aqua (Water) to 100%, Acrylates Copolymer (PAC4) 2.2%, Sodium Coco-Glucoside Sulfosuccinate 1.3%, Sodium Coco-Glucoside Tartrate 5.7% Cocamidopropyl Betaine 2%, PEG-7 Glyceryl Cocoate 1.0%, Aloe Barbadensis Leaf Juice 0.5%, Preservative 0.5%, Sodium Hydroxide to pH 6.5-7.0.

**Example 14**

Antidandruff Conditioning Shampoo

Phase A: Aqua (Water) to 100%, Acrylates Copolymer (PAC4) 1.5%, Sodium Laureth Sulfate 4.8%, Sodium Lauroyl Sulfate 5.0% Sodium Hydroxide to pH 6.5-7.0, Glycerin 2.0%, Cocamidopropyl Betaine 1.35%, Zinc Thiosalycylate 1.0%.
Phase B: Aqua (Water) 20%, Guar hydroxypropyltrimonium Chloride 0.5%
Phase C: P Fragrance 0.5%, Preservative 0.5%, Colour q.s., Sodium Chloride 0.2, Citric Acid Solution to pH 5.0-5.5

**Example 15**

Bath Gel with Beads

Phase A: Aqua (Water) to 100%, Disodium EDTA 0.1%, Acrylates Copolymer (PAC4) 2.7%, Sodium Laureth Sulfate 9%, Sodium Hydroxide to pH 6.5-7.0, Propylene Glycol 2.0%, Cocamidopropyl Betaine 1.2%,
**[0134]** Phase B: Aqua (Water) 5%, Benzophenone-4 0.02, Preservative 0.5%, Jojoba Esters 0.2%

**Example 16**

Foundation cream

Phase A: Isopropyl Isostearate 5.0%, Cetyl Hexylhexanoate 5.0%, Cetearyl Alcohol 2.0%, Cetearyl Alcohol 2.0%
Phase B: Titanium Dioxide 8.0%, Yellow Iron Oxide 0.6%, Red Iron Oxide 0.2%, Propylene Glycol 2.0%, Butylene Glycol 2.0%
Phase C: Aqua (Water) to 100%, PEG-20 Methyl Glucose Sesquistearate 1.8%
Phase D: Acrylates Copolymer (PAC4) 0.9%, Triethanolamine 0.9%, Preservative 0.3

**Example 17**

Facial cream

Phase A: Aqua (Water) to 100%, Glycerin 1.3%, Isopropyl Isostearate 1.75, Isopropyl Palmitate 4.0%, Cetyl Alcohol 1.5%, Cetearyl Alcohol and Ceteareth-20 3.0%
Phase B: Acrylates Copolymer (PAC4) 1.5%, Sodium Hydroxide to pH 6.5-7.0, Preservative

**Example 18**

Milky Shower gel

Phase A: Aqua (Water) to 100%, Acrylates Copolymer (PAC4) 1.5%,Sodium Laureth Sulfate 7.5%, Sodium Dilaureth-7 Citrate 3.75%, Cocamidopropyl Betaine 2.4%, Sodium Hydroxide to pH 6.5-7.0
Phase B: Aqua (Water) 5.0%, Styrene/Acrylates Copolymer 0.45%, Preservative 0.5, Sodium Chloride 1.0%

**Example 19**

Revitalizing Cream

Phase A: Aqua (Water) to 100%, Glycerin 1.3%, Isocetyl Isostearate 2.0%, Helianthus Annus Seed Oil 3%, Cethyl Alcohol 1.5%, Glyceril Stearate 2%, PEG100 Stearate 2.0%
Phase B: Acrylate Copolymer (PAC4) 1.5%, Sodium Hydroxide 0.95%, Cyclopentasiloxane 3%, Preservative 0.5%

**Example 20**

Shower Gel

Phase A: Aqua (Water) to 100%, Disodium EDTA 0.1%, Lithium Magnesium Sodium Silicate 2%, Acrylate Copolymer (PAC4) 1.5%, Sodium Hydroxide to pH 6.5-7.0, Sodium Lauroyl Sarcosinate 4.5%, Cocamide DEA 4%, Sodium Cocoamphoacetate 3%, Preservative 0.1%

**Example** 21

Conditioning 2-in-1 Shampoo

Phase A: Aqua (Water) to 100%, Acrylates Copolymer (PAC4) 1.5%,Sodium Laureth Sulfate 9%, Sodium Hydroxide to pH 6.5-7.0, Cocamidopropyl Hydroxysultanine 5%, Disodium Laureth Sulfoccinate 4%
Phase B: Dimethicone 3%,Preservative 0.5%, Yellow 100.1%, Citric Acid 0.4%

**Example 22**

Shampoo for Men

Phase A: Aqua (Water) to 100%, Sodium Laureth Sulfate 10%, Cocoglucoside 5%, Laureth-7 Citrate 2.0%, Acrylates Copolymer (PAC4) 1%, Sodium Hydroxide to pH 6.5-7.0
Phase B: Hydrolyzed Wheat Protein 0.5%, Aloe Barbadensis Leaf Extract 0.1%, Dicaprylyl Ether 0.5%, Magnesium Chloride 0.5%, Sodium Chloride 2%.

**Example 23**

Extra nourishing body wash

Phase A: Aqua (Water) to 100%, Tetrasodium EDTA 0.05%, Ammonium Lauryl Sulfate 4.5%, Ammonium Laureth Sulfate 7.5%, Heliantus Annuus Seed Oil 18%, Acrylates Copolymer (PAC4) 3%
Phase B: Guar Hydroxypropyltrimonium Chloride 0.3%, Aqua (Water) 5%, Cucumis Sativa Fruit Extract 0.2%, Preservative 0.5%

**Example 24**

Hand Sanitizer

Phase A: Aqua (Water) to 100%, Acrylates Copolymer (PAC4) 2.1%, Triethanolamine to pH 7.0, Alcohol 65%, PEG75 Lanolin 0.25%, Propylene Glycol 0.5%
Phase B: Polysorbate 20 1.0, PEG40 Hydrogenated Castor Oil 1.0, Citrus Medica Lemon Oil 1.0%

### Example 25

Strong Hold Hair Gel

Phase A: Aqua (Water) to 100%, Acrylates Copolymer (PAC4) 2.0%, Triethanolamine 0.75%, PVP/VA 6%
Phase B: Aqua (Water) 5.0%, Disodium EDTA 0.05%, DMDM Hydantoin 0.20%

### Example 26

Brown Hair Color Shampoo

Phase A: Aqua (Water) to 100%, Acrylates Copolymer (PAC4) 3.0%, Butylene Glycol 5.0%, Sodium Cocoamphoacetate 5.50%" Cocamidopropyl Betaine 1.0%, Decyl Glucoside 2.0%, Sodium Hydroxide to pH 7.0.
Phase B: Aqua (Water) 5%, Cl 12251 0.25%, Cl56059 0.125%, Cl 12245 0.125%, PEG-12 Dimethicone 0.20%

### Example 27

Antibacterial Hand Soap

Phase A: Aqua (Water) to 100%, Acrylates Copolymer (PAC4) 2.5%, Ammonium Lauryl Sulfate 7.5%, Disodium Laureth Sulfosuccinate 3.2%, Cocamidopropyl Betaine 1.5%, Triethanolamine 0.8%
Phase B: Glycerin 2.0%, Propylene Glycol 2.0%, Triclosan 0.5%, DMDM Hydantoin 0.30%

### Example 28

Soap Based Facial Cleanser

Phase A: Lauric Acid 10%, Myristic Acid 5.0%, Stearic Acid 15.0%, Glyceryl Monostearate 2.0%
Phase B: Aqua (Water) to 100%, Potassium Hydroxide 6.2%, Glyceryn 20.0%, Propylene Glycol 10.0%, Sodium Methyl Cocoyl Taurate 0.9%, PEG-90 Diisostearate 1.0%
Phase C: Aqua (Water) 10%, Acrylates Copolymer (PAC4) 1.5%

### Claims

1. Personal care compositions comprising:

    i) from 0.2 to 10 % by weight of a thickening agent which is a crosslinked alkali swellable polyacrylate obtainable by polymerization of:

        a) from 20 to 70% by weight of a monoethylenically unsaturated monomer containing a carboxylic group;
        b) from 20 to 70% by weight of a (meth)acrylic acid ester;
        c) from 0.05 to 3% by weight of an unsaturated monomer containing one or more acetoacetyl or cyanoacetyl groups;
        d) from 0.01 to 3% by weight of a polyethylenically unsaturated monomer;
        e) from 0 to 10% by weight of a nonionic acrylic associative monomer;

    ii) from 0.1 to 60% by weight of a surfactant selected from anionic surfactants, amphoteric surfactants, cationic surfactants, zwitterionic surfactants, non-ionic surfactants, and mixture thereof.

2. Personal care compositions according to claim 1 comprising: from 0.5 to 20 % by weight of the surfactant.

3. Personal care compositions according to claim 2 comprising: from 5 to 20 % by weight of the surfactant.

4. Personal care compositions according to claim 1 in which the unsaturated monomer containing one or more acetoacetyl or cyanoacetyl groups is an acetoacetoxyalkyl (meth)acrylate) of formula (I)

$$CH_2=CR-CO-OR_1- O-CO-CH_2-CO-CH_3 \text{ (I)}$$

in which R is H or $CH_3$ and $R_1$ is a $C_2$-$C_4$ linear or branched alkylene radical group.

5. Personal care compositions according to claim 4 in which in formula (I) R is $CH_3$ and $R_1$ is the ethylene radical.

6. Personal care compositions according to claim 1 in which the monoethylenically unsaturated monomer containing a carboxylic group is acrylic acid, methacrylic acid, itaconic acid or mixture thereof and the (meth)acrylic acid ester is a $C_1$-$C_{40}$ (meth)acrylic acid alkyl ester.

7. Personal care compositions according to claim 6 in which the monoethylenically unsaturated monomer containing a carboxylic group is methacrylic acid and the (meth)acrylic acid ester is ethyl acrylate.

8. Personal care compositions according to claim 1 in which the crosslinked alkali swellable polyacrylate is obtainable by polymerization of:

   a) from 20 to 70% by weight of a monoethylenically unsaturated monomer containing a carboxylic group;
   b) from 20 to 70% by weight of a (meth)acrylic acid ester;
   c) from 0.1 to 0.5% by weight of an unsaturated monomer containing one or more acetoacetyl or cyanoacetyl groups;
   d) from 0.01 to 3% by weight of a polyethylenically unsaturated monomer;
   e) from 1.0 to 2.0% by weight of a nonionic acrylic associative monomer.

9. Personal care compositions according to any of the claims from 1 to 8 which are shampoos or skin and body cleansers.

10. Personal care compositions according to claim 9 which are shampoos selected among 2-in-1 shampoos, baby shampoos, conditioning shampoos, bodifying shampoos, moisturizing shampoos, temporary hair color shampoos, 3-in-1 shampoos, anti-dandruff shampoos, hair color maintenance shampoos, acid or neutralizing shampoos, salicylic acid shampoos, or are skin and body cleansers selected among shower gels, bath foams, facial cleansers, intimate cleansers, moisturizing body washes, antibacterial body washes, bath gels, hand soaps, bar soaps, body scrubs, bubble baths, facial scrubs, foot scrubs.

11. Personal care compositions according to any of the claims from 1 to 8 which are hair or skin care compositions.

12. Personal care compositions according to claim 11 which are hair care compositions selected among hair dyes, hair conditioners, hair creams and hair styling formulations, or are skin care compositions selected among skin lotions, balms or creams, alpha-hydroxy acid lotions and creams, beta-hydroxy acid creams and lotions, skin whiteners, self tanning lotions, sunscreen lotions, barrier lotions, moisturizers, vitamin C creams, antibacterial lotions and other moisturizing lotions and creams, liquid talc products, skin gels, facial masks, body masks, hydroalcoholic gels, body gels, sunscreen gels, make up foundations, sun care formulations, antiperspirants.

13. Personal care compositions according to any of the claims from 1 to 12 additionally comprising one or more additives selected among silicones and water insoluble oily compounds, cationic polymers, cosmetically acceptable mediums, other natural or synthetic thickeners, skin and hair care actives, particulate materials, perfumes, fragrances, essential oils, dyes, pearlescent agents, opacifiers, scrubbing agents, enzymes, preservatives, disinfecting agents, antimicrobial additives, foaming agents, anti-foam agents, humectants, moisturizing agents, chelating agents, emollients, conditioning agents, whitening agents, solvents and pH buffering means.

**Patentansprüche**

1. Körperpflegezusammensetzungen, umfassend:

   i) 0,2 bis 10 Gewichts-% eines Verdickungsmittels, das ein vernetztes, in Alkali quellbares Polyacrylat ist, das erhalten werden kann durch Polymerisation von:

   a) 20 bis 70 Gewichts-% eines monoethylenisch ungesättigten Monomers, das eine Carboxylgruppe enthält;
   b) 20 bis 70 Gewichts-% eines (Meth)acrylsäureesters;
   c) 0,05 bis 3 Gewichts-% eines ungesättigten Monomers, das eine oder mehrere Acetoacetyl- oder Cyanoacetylgruppen enthält;

d) 0,01 bis 3 Gewichts-% eines polyethylenisch ungesättigten Monomers;

e) 0 bis 10 Gewichts-% eines nichtionischen acrylischen assoziativen Monomers;

ii) 0,1 bis 60 Gewichts-% eines Tensids, das aus anionischen Tensiden, amphoteren Tensiden, kationischen Tensiden, zwitterionischen Tensiden, nichtionischen Tensiden und Mischungen davon ausgewählt ist.

2. Körperpflegezusammensetzungen nach Anspruch 1, umfassend:

0,5 bis 20 Gewichts-% des Tensids.

3. Körperpflegezusammensetzungen nach Anspruch 2, umfassend:

5 bis 20 Gewichts-% des Tensids.

4. Körperpflegezusammensetzungen nach Anspruch 1, wobei das ungesättigte Monomer, das eine oder mehrere Acetoacetyl- oder Cyanoacetylgruppen enthält, ein Acetoacetoxyalkyl(meth)acrylat) der folgenden Formel (I) ist:

$$CH_2=CR-CO-O-R_1-O-CO-CH_2-CO-CH_3 \qquad (I)$$

wobei R H oder $CH_3$ ist, und $R_1$ eine lineare oder verzweigte $C_2$-$C_4$-Alkylenradikalgruppe ist.

5. Körperpflegezusammensetzungen nach Anspruch 4, wobei in Formel (I) R $CH_3$ ist, und $R_1$ das Ethylenradikal ist.

6. Körperpflegezusammensetzungen nach Anspruch 1, wobei das monoethylenisch ungesättigte Monomer, das eine Carboxylgruppe enthält, Acrylsäure, Methacrylsäure, Itaconsäure oder eine Mischung davon ist, und der (Meth)acryl-säureester ein $C_1$-$C_{40}$-(Meth)acrylsäurealkylester ist.

7. Körperpflegezusammensetzungen nach Anspruch 6, wobei das monoethylenisch ungesättigte Monomer, das eine Carboxylgruppe enthält, Metharylsäure ist, und der (Meth)acrylsäureester Ethlyacrylat ist.

8. Körperpflegezusammensetzungen nach Anspruch 1, wobei das vernetzte, in Alkali quellbare Polyacrylat erhalten werden kann durch Polymerisation von:

a) 20 bis 70 Gewichts-% eines monoethylenisch ungesättigten Monomers, das eine Carboxylgruppe enthält;

b) 20 bis 70 Gewichts-% eines (Meth)acrylsäureesters;

c) 0,1 bis 0,5 Gewichts-% eines ungesättigten Monomers, das eine oder mehrere Acetoacetyl- oder Cyanoacetylgruppen enthält;

d) 0,01 bis 3 Gewichts-% eines polyethylenisch ungesättigten Monomers;

e) 1,0 bis 2,0 Gewichts-% eines nichtionischen acrylischen assoziativen Monomers.

9. Körperpflegezusammensetzungen nach einem der Ansprüche 1 bis 8, wobei es sich um Shampoos oder Haut- und Körperreinigungsmittel handelt.

10. Körperpflegezusammensetzungen nach Anspruch 9, wobei es sich um Shampoos, die unter 2-in-1-Shampoos, Baby-Shampoos, Konditionierungsshampoos, Bodifying-Shampoos, feuchtigkeitsspendenden Shampoos, Shampoos für temporäre Haarfärbung, 3-in-1-Shampoos, Antischuppenshampoos, Haarfarberhaltungsshampoos, säurehaltigen oder neutralisierenden Shampoos, salicylsäurehaltigen Shampoos ausgewählt sind, oder Haut- und Körperreinigungsmittel handelt, die unter Duschgelen, Badeschäumen, Gesichtsreinigern, Reinigungsmitteln für den Intimbereich, feuchtigkeitsspendenden Waschlotionen für den Körper, antibakteriellen Waschlotionen für den Körper, Badegelen, Handseifen, Festseifen, Körperpeelings, Schaumbädern, Gesichtspeelings, Fußpeelings ausgewählt sind.

11. Körperpflegezusammensetzungen nach einem der Ansprüche 1 bis 8, wobei es sich um Haar- oder Hautpflegezusammensetzungen handelt.

12. Körperpflegezusammensetzung nach Anspruch 11, wobei es sich um Haarpflegezusammensetzungen, die unter Haarfärbemitteln, Haarkonditionierern, Haarcremes und Haarstyling-Formulierungen ausgewählt sind, oder Haupt-

**EP 2 689 000 B1**

pflegezusammensetzungen handelt, die unter Hautlotionen, -balsamen oder -cremes, alpha-hydrozylsäurehaltigen Lotionen oder Cremes, beta-hydromylsäurehaltigen Cremes und Lotionen, Hautaufhellern, Selbstbräunern, Sonnenschutzlotionen, Barrierelotionen, Feuchtigkeitscremes, Vitamin-C-Cremes, antibakteriellen Lotionen und anderen feuchtigkeitsspendenden Lotionen und Cremes, flüssigen Talkprodukten, Hautgelen, Gesichtsmasken, Körpermasken, hydroalkoholischen Gelen, Körpergelen, Sonnenschutzgelen, Make-up-Grundierungen, Sonnenschutzformulierungen, Deodorants ausgewählt sind.

**13.** Körperpflegezusammensetzungen nach einem der Ansprüche 1 bis 12, zusätzlich umfassend ein oder mehrere Additive, die unter Silikonen und wasserunlöslichen öligen Verbindungen, kationischen Polymeren, kosmetisch unbedenklichen Medien, anderen natürlichen oder synthetischen Verdickungsmitteln, aktiven Haut- und Haarpflegesubstanzen, Schwebstoffen, Parfümen, Duftstoffen, ätherischen Ölen, Farbstoffen, Perlglanzmitteln, Trübungsmitteln, Reinigungsmitteln, Enzymen, Konservierungsmitteln, Desinfektionsmitteln, antimikrobiellen Additiven, Schäummitteln, Antischaummitteln, Feuchthaltemitteln, feuchtigkeitsspendenden Mitteln, Chelatbildnern, Erweichungsmitteln, Konditionierungsmitteln, Aufhellungsmitteln, Lösungsmitteln und pH-Puffermitteln ausgewählt sind.

## Revendications

**1.** Compositions de soin personnel comprenant :

I) entre 0,2 et 10 % en poids d'un agent épaississant qui est un polyacrylate réticulé gonflable en présence d'un alcali, obtenu par polymérisation de :

a) entre 20 et 70 % en poids d'un monomère monoéthyléniquement insaturé contenant un groupe carboxylique ;
b) entre 20 et 70 % en poids d'un ester d'acide (méth)acrylique ;
c) entre 0,05 et 3 % en poids d'un monomère insaturé contenant un ou plusieurs groupe(s) d'acétoacétyle ou de cyanoacétyle ;
d) entre 0,01 et 3 % en poids d'un monomère polyéthyléniquement insaturé ;
e) entre 0 et 10 % en poids d'un monomère acrylique non ionique associatif;

II) entre 0,1 et 60 % en poids d'un tensioactif sélectionné parmi des tensioactifs anioniques, des tensioactifs amphotères, des tensioactifs cationiques, des tensioactifs zwitterioniques, des tensioactifs non-Ioniques et un mélange de ces tensioactifs.

**2.** Compositions de soin personnel selon la revendication 1 comprenant : entre 0,5 et 20 % en poids de tensioactif.

**3.** Compositions de soin personnel selon la revendication 2 comprenant : entre 5 et 20 % en poids de tensioactif.

**4.** Compositions de soin personnel selon la revendication 1 dans lesquelles le monomère insaturé contenant un ou plusieurs groupe(s) d'acétoacétyle ou de cyanoacétyle est un (méth)acrylate d'acétoacétoxyalkyle de formule (I)

$$CH_2=CR-CO-O-R_1 - O-CO-CH_2-CO-CH_3 \qquad (I)$$

où R correspond à H ou $CH_3$ et $R_1$ est un groupe radical $C_2$-$C_4$ alkylène linéaire ou ramifié.

**5.** Compositions de soin personnel selon la revendication 4 dans lesquelles, dans la formule (I), R correspond à $CH_3$ et $R_1$ est un radical éthylène.

**6.** Compositions de soin personnel selon la revendication 1 dans lesquelles le monomère monoéthyléniquement insaturé contenant un groupe carboxylique est un acide acrylique, un acide méthacrylique, un acide itaconique ou un mélange de ces acides et l'ester d'acide (méth)acrylique est un alkylester $C_1$-$C_{40}$ de l'acide (méth)acrylique.

**7.** Compositions de soin personnel selon la revendication 6 dans lesquelles le monomère monoéthyléniquement insaturé contenant un groupe carboxylique est un acide méthacrylique et l'ester d'acide (méth)acrylique est l'acrylate d'éthyle.

**8.** Compositions de soin personnel selon la revendication 1 dans lesquelles le polyacrylate réticulé gonflable en présence d'un alcali est obtenu par polymérisation de :

a) entre 20 et 70 % en poids d'un monomère monoéthyléniquement insaturé contenant un groupe carboxylique;
b) entre 20 et 70 % en poids d'un ester d'acide (méth)acrylique ;
c) entre 0,1 et 0,5 % en poids d'un monomère insaturé contenant un ou plusieurs groupe(s) d'acétoacétyle ou de cyanoacétyle ;
d) entre 0,01 et 3 % en poids d'un monomère polyéthyléniquement insaturé ;
e) entre 1,0 et 2,0 % en poids d'un monomère acrylique non ionique associatif.

**9.** Compositions de soin personnel selon l'une quelconque des revendications 1 à 8 constituant des shampooings ou des produits nettoyants pour la peau et le corps.

**10.** Compositions de soin personnel selon la revendication 9 (constituant) qui sont des shampooings sélectionnés parmi les shampooings 2-en-1, les shampooings pour bébés, les shampooings conditionnants, les shampooings volumateurs, les shampooings hydratants, les shampooings pour cheveux colorés, les shampooings 3-en-1, les shampooings antipelliculaires, les shampooings protecteurs de couleur, les shampooings acides ou neutralisants, les shampooings d'acide salicylique ou sont des nettoyants pour la peau et pour le corps sélectionnés parmi les gels douche, les bains moussants, les savons pour le visage, les savons de toilette intime, les lotions hydratantes pour le corps, les lotions antibactériennes pour le corps, les gels pour le bain, les savons pour les mains, les pains de savon, les gommages pour le corps, les bains à bulles, les gommages pour le visage, les gommages pour les pieds.

**11.** Compositions de soin personnel selon l'une quelconque des revendications 1 à 8 qui sont des compositions de soin pour les cheveux ou la peau.

**12.** Compositions de soin personnel selon la revendication 11 qui sont des compositions de soin pour les cheveux sélectionnées parmi les colorants pour cheveux, les après-shampooings, les crèmes pour cheveux et les formules coiffantes ou sont des compositions de soin pour la peau sélectionnées parmi les lotions pour la peau, les baumes ou les crèmes, les lotions et crèmes à base d'acide alpha-hydroxylé, les crèmes et lotions à base d'acide bêta-hydroxy, les agents blanchissants pour la peau, les lotions auto-bronzantes, les crèmes solaires, les lotions barrière, les crèmes hydratantes, les crèmes à base de vitamine C, les lotions antibactériennes et autres lotions et crèmes hydratantes, les produits à base de talc liquide, les gels pour la peau, les masques pour visage, les masques pour le corps, les gels hydroalcooliques, les gels pour le corps, les gels solaires, les bases pour maquillage, les formules de soin solaire, les antitranspirants.

**13.** Compositions de soin personnel selon l'une quelconque des revendications 1 à 12 comprenant de plus un ou plusieurs additifs sélectionnés parmi les silicones et les composés huileux insolubles dans l'eau, les polymères cationiques, les milieux cosmétiquement acceptables, d'autres épaississeurs naturels ou synthétiques, les actifs de soin de la peau et des cheveux, les particules, les parfums, les fragrances, les huiles essentielles, les colorants, les nacres, les opacifiants, les agents gommants, les enzymes, les agents conservateurs, les agents désinfectants, les additifs antimicrobiens, les agents moussants, les agents anti-mousse, les humectants, les agents d'hydratation, les agents de chélation, les émollients, les agents de conditionnement, les agents blanchissants, les solvants et les dispositifs tampons de pH.

**EP 2 689 000 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 870994 A
- EP 13836 A
- US 4529773 A
- US 4138381 A
- US 6140438 A
- EP 1272159 A
- US 5516453 A
- US 4325856 A
- US 4654397 A
- US 4814373 A